# EUROPEAN PATENT APPLICATION

(11) **EP 4 360 622 A1**
(43) Date of publication of application: **01.05.2024**
(21) Application number: 22827682.0
(22) Date of filing: 24.06.2022
(51) Int. Cl.: A61K 9/08, A61K 31/407, A61K 47/18, A61P 29/00

(54) **KETOROLAC LIQUID COMPOSITION, PREPARATION METHOD THEREFOR, AND APPLICATION THEREOF**

(30) Priority: 24.06.2021 CN 202110702722; 24.06.2021 CN 202110702691
(71) Applicant: Shanghai Aurora Biotechnology Co., Ltd., Shanghai 201210 (CN)
(72) Inventor: CHEN, Zhixiang, Shanghai 201210 (CN); CHEN, Bangyin, Shanghai 201210 (CN); LIU, Shuang, Shanghai 201210 (CN); GUO, Zhen, Shanghai 201210 (CN); FU, Jun, Shanghai 201210 (CN); WANG, Tingting, Shanghai 201210 (CN); YING, Shuhuan, Shanghai 201210 (CN)
(74) Representative: Gille Hrabal Partnerschaftsgesellschaft mbB Patentanwälte
(86) International application number: PCT/CN2022/100988
(87) International publication number: WO 2022/268190

(57) **Abstract**

A ketorolac liquid composition, a preparation method therefor, and an application thereof. Provided is a ketorolac liquid composition, comprising the following components: ketorolac tromethamine, a stabilizer, a buffer, an osmotic pressure regulator, and a pH regulator; the ketorolac liquid composition does not contain ethanol; a concentration of ketorolac tromethamine is 0.15 mmol/L-160.00 mmol/L, and the concentration refers to a ratio of a molar amount of the ketorolac tromethamine to a volume of the ketorolac liquid composition. The ketorolac liquid composition does not contain an organic solvent, has stable physical and chemical properties, has less in-vivo irritation, has a lower adverse reactions, is safer and more convenient, can improve medication compliance of patients and the convenience of clinical administration, and has good market prospects.

## Description

The present disclosure claims priority to the prior application with the patent application No. 202110702722.1 and entitled "KETOROLAC LIQUID COMPOSITION, PREPARATION METHOD THEREFOR, AND APPLICATION THEREOF" filed to the China National Intellectual Property Administration on June 24, 2021, and the prior application with the patent application No. 202110702691.X and entitled "KETOROLAC LIQUID COMPOSITION, PREPARATION METHOD THEREFOR, AND APPLICATION THEREOF" filed to the China National Intellectual Property Administration on June 24, 2021, which are incorporated herein by reference in their entirety.

### TECHNICAL FIELD

The present disclosure relates to a ketorolac liquid composition, a preparation method therefor and use thereof, belonging to the field of pharmaceutical compositions.

### BACKGROUND

Ketorolac is a piromidic acid derivative non-steroidal anti-inflammatory medicament, being a prostaglandin synthase inhibitor with potent analgesic effect and moderate anti-inflammatory and antipyretic effects, and acts mainly by blocking the cyclooxygenase of arachidonic acid metabolism to reduce prostaglandin synthesis.

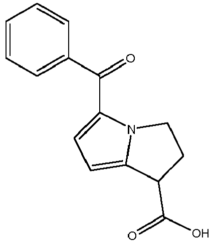

Ketorolac tromethamine, a salt form of ketorolac, is a novel NSAID drug that can be administered by injection, developed by Syntex Corporation in the United States and first launched in Italy in 1990. The analgesic effect of ketorolac tromethamine is stronger than its anti-inflammatory effect, and a plurality of research results show that ketorolac tromethamine is superior to morphine in relieving postsurgical pain. Ketorolac tromethamine is mainly used in the treatment of postsurgical and postpartum severe pain or pain in the middle and late stages of cancer, and externally in moderate or severe pain, and the like in clinic. In standard animal models for analgesic effect, the analgesic activity of this medication is 800 times that of aspirin, stronger than that of indomethacin and naproxen, and equivalent to or better than that of phenylbutazone. In multiple tests, the anti-inflammatory activity of this medication is equivalent to or stronger than that of indomethacin, stronger than that of naproxen, and significantly better than that of phenylbutazone. This drug has the advantages of no toxic and side effects such as central nervous system damage and addiction, and no adverse effects such as respiratory depression or constipation.

At present, ketorolac tromethamine used clinically mainly includes oral administration preparations and injection administration preparations, such as tablets, capsules, injections, and the like. Among them, ketorolac tromethamine injection is widely used due to its fast onset of action. However, the current original injection contains ethanol, and injections containing organic vehicles such as ethanol have a certain degree of toxicity, which can easily cause irritation upon injection, thereby reducing patient compliance. In addition, ketorolac tromethamine injection is often used in combination with morphine and other injections in clinical practice, and ethanol has a certain influence on the absorption and metabolism of morphine, increasing the probability of adverse effects, so ketorolac tromethamine injection containing ethanol carries an increased risk of adverse effects. In addition, the injection can generate white dots or crystals continuously during long-term storage, affecting visible foreign matters and insoluble particles, and also posing certain safety hazards.

Therefore, the search for ketorolac dosage forms which have stable physical and chemical properties, have less *in-vivo* irritation, have fewer adverse effects, are safer and more convenient, and have improved medication compliance of patients is an urgent technical problem that needs to be addressed at present.

### SUMMARY

In order to ameliorate the technical problem described above, the present disclosure provides a ketorolac liquid composition, comprising the following components: ketorolac or a pharmaceutically acceptable salt thereof, a stabilizer, an osmolarity regulator, a pH regulator, and optionally a buffer present or absent,
wherein the ketorolac liquid composition does not comprise ethanol; a concentration of ketorolac or the pharmaceutically acceptable salt thereof is 0.15 mmol/L-160.00 mmol/L, and the concentration refers to the ratio of the molar amount of ketorolac or the pharmaceutically acceptable salt thereof to the volume of the ketorolac liquid composition.

According to an embodiment of the present disclosure, in the ketorolac liquid composition, the pharmaceutically acceptable salt of ketorolac is preferably ketorolac tromethamine.

According to an embodiment of the present disclosure, in the ketorolac liquid composition, the concentration of ketorolac or the pharmaceutically acceptable salt thereof (e.g., ketorolac tromethamine) is preferably 0.15 mmol/L-0.38 mmol/L, or 30.00 mmol/L-160.00 mmol/L, such as 39.50 mmol/L-80.00 mmol/L, an example of which may be 39.85 mmol/L or 79.70 mmol/L. According to an embodiment of the present disclosure, in the ketorolac liquid composition, the stabilizer is preferably one or more selected from lysine, arginine, meglumine, phosphoric acid, hydroxypropyl-β-cyclodextrin and sulfobutyl-β-cyclodextrin, and further preferably one or more selected from arginine, meglumine, hydroxypropyl-β-cyclodextrin and sulfobutyl-β-cyclodextrin. The lysine may be L-lysine and/or D-lysine. The arginine may be L-arginine and/or D-arginine. According to an embodiment of the present disclosure, in the ketorolac liquid composition, the molar ratio of ketorolac or the pharmaceutically acceptable salt thereof to the stabilizer is preferably 1:0.005-1:30, and further preferably 1:0.05-1:5, such as 1:0.1, 1:0.2, 1:0.3, 1:0.4, 1:0.5, 1:1, 1:1.5, 1:2, 1:2.5, 1:3, 1:3.5, 1:4, 1:4.5, or 1:5.

According to an embodiment of the present disclosure, in the ketorolac liquid composition, the buffer is preferably one or more selected from acetic acid and a salt thereof, boric acid and a salt thereof, citric acid and a salt thereof, tartaric acid and a salt thereof, oxalic acid and a salt thereof, carbonic acid and a salt thereof, and tromethamine and a salt thereof, and further preferably one or more selected from citric acid and a salt thereof and acetic acid and a salt thereof, wherein the salt may be a sodium salt, a potassium salt, a calcium salt, an ammonia salt, an ammonium salt, or the like.

According to an embodiment of the present disclosure, in the ketorolac liquid composition, the molar ratio of ketorolac or the pharmaceutically acceptable salt thereof to the buffer is preferably 1:0-1:30, and further preferably 1:0-1:5. When a buffer is present in the ketorolac liquid composition, the molar ratio of ketorolac or the pharmaceutically acceptable salt thereof to the buffer may be 1:0.1, 1:0.2, 1:0.3, 1:0.4, 1:0.5, 1:1, 1:1.5, 1:2, 1:2.5, 1:3, 1:3.5, 1:4, 1:4.5, or 1:5. According to an embodiment of the present disclosure, in the ketorolac liquid composition, the pH regulator is preferably one or more selected from hydrochloric acid, sulfuric acid, acetic acid, citric acid, sodium hydroxide, sodium bicarbonate and aqueous ammonia.

According to an embodiment of the present disclosure, the pH of the ketorolac liquid composition is preferably in a range of 6.0-9.0, and further preferably in a range of 6.5-8.5, such as 7.0-8.0, e.g., 7.2-7.6, an example of which may be 7.4.

According to an embodiment of the present disclosure, in the ketorolac liquid composition, the osmolarity regulator is preferably one or more selected from sodium chloride, mannitol, glucose and lactose.

According to an embodiment of the present disclosure, the osmolarity of the ketorolac liquid composition is preferably in a range of 200 mOsmol/kg-500 mOsmol/kg, and further preferably in a range of 260 mOsmol/kg-330 mOsmol/kg.

According to an embodiment of the present disclosure, the ketorolac liquid composition further comprises water, such as water for injection.

According to an embodiment of the present disclosure, the ketorolac liquid composition may be in the form of an oral liquid or an injection.

The present disclosure further provides a preparation method for the ketorolac liquid composition, comprising mixing the components described above.

According to an embodiment of the preparation method of the present disclosure, the preparation method further comprises sterilizing the mixed components.

The present disclosure further provides a lyophilizate which is manufactured from the injection, preferably by a lyophilization process.

The present disclosure further provides use of the ketorolac liquid composition for manufacturing a medicament for the prevention and/or treatment of inflammation, such as for manufacturing an anti-inflammatory medicament, preferably a non-steroidal anti-inflammatory medicament.

The present disclosure further provides a method for preventing and/or treating inflammation by providing a therapeutically effective amount of the ketorolac liquid composition to a patient in need thereof.

The preferred conditions described above may be combined arbitrarily to give preferred embodiments of the present disclosure without departing from the general knowledge in the art.

The reagents and starting materials used in the present disclosure are commercially available.

The positive progress effects of the present disclosure are as follows: The ketorolac liquid composition of the present disclosure improves the defects of ketorolac preparations in the prior art, such as poor physical and chemical stability, relatively high *in-vivo* irritation, poor medication compliance of patients, low safety, and the like. The ketorolac liquid composition does not comprise an organic solvent, has stable physical and chemical properties, has less *in-vivo* irritation, has lower incidence of adverse effects, and is safer. In addition, the ketorolac liquid composition is more convenient for administration, and can improve medication compliance of patients and the convenience of clinical medication, thus having good market prospects.

### BRIEF DESCRIPTION OF THE DRAWINGS

FIG. 1 shows the mean plasma concentration-time curves of the samples of Comparative Example 9 and Example 2 after single intravenous injection at 1.5 mg/kg in SD rats (N = 3/time point);
FIG. 2 shows the mean plasma concentration-time curves of the samples of Comparative Example 10 and Example 3 after single intramuscular injection at 6 mg/kg in SD rats (N = 3/time point).

### DETAILED DESCRIPTION

The present disclosure is further illustrated by the following examples. However, the protection scope of the present disclosure is not limited to the examples described below.

Experimental procedures without specified conditions in the following examples are conducted in accordance with conventional procedures and conditions, or in accordance with the manufacturer's manual.

### Example 1

### Formula:

| | |
|---|---|
| Ketorolac tromethamine (79.70 mmol/L) | 1.35g |
| Sulfobutyl-β-cyclodextrin (27.74 mmol/L) | 2.7g |
| Sodium chloride | 0.0027g |
| Sodium hydroxide | Adjusting the pH to about 7.4 |
| Water for injection | Added to 45 mL |

### Preparation process:

1) Sulfobutyl-β-cyclodextrin and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.5 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Example 2

### Formula:

| | |
|---|---|
| Ketorolac tromethamine (39.85 mmol/L) | 450g |
| Sulfobutyl-β-cyclodextrin (18.49 mmol/L) | 1200g |
| Sodium chloride | 137.4g |
| Sodium hydroxide | Adjusting the pH to about 7.4 |
| Water for injection | Added to 30000 mL |

### Preparation process:

1) Sulfobutyl-β-cyclodextrin and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.5 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Example 3

### Formula:

| | |
|---|---|
| Ketorolac tromethamine (79.70 mmol/L) | 900g |
| Sulfobutyl-β-cyclodextrin (36.99 mmol/L) | 2400g |
| Sodium chloride | 1.8g |
| Sodium hydroxide | Adjusting the pH to about 7.4 |
| Water for injection | Added to 30000 mL |

### Preparation process:

1) Sulfobutyl-β-cyclodextrin and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.5 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Example 4

### Formula:

| | |
|---|---|
| Ketorolac tromethamine (79.70 mmol/L) | 1.35g |
| Sulfobutyl-β-cyclodextrin (46.23 mmol/L) | 4.5g |
| Sodium chloride | 0.0027g |
| Sodium hydroxide | Adjusting the pH to about 7.4 |
| Water for injection | Added to 45 mL |

### Preparation process:

1) Sulfobutyl-β-cyclodextrin and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.5 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Example 5

### Formula:

| | |
|---|---|
| Ketorolac tromethamine (79.70 mmol/L) | 1.2g |
| Hydroxypropyl-β-cyclodextrin (64.87 mmol/L) | 4g |
| Sodium chloride | 0.1344g |
| Sodium hydroxide | Adjusting the pH to about 7.4 |
| Water for injection | Added to 40 mL |

### Preparation process:

1) Hydroxypropyl-β-cyclodextrin and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.1 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Example 6

### Formula:

| | |
|---|---|
| Ketorolac tromethamine (79.70 mmol/L) | 1.2g |
| Arginine (24.11 mmol/L) | 0.168g |
| Sodium chloride | 0.174g |
| Hydrochloric acid | Adjusting the pH to about 7.4 |
| Water for injection | Added to 40 mL |

### Preparation process:

1) Arginine and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.2 mol/L hydrochloric acid solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Example 7

### Formula:

| | |
|---|---|
| Ketorolac tromethamine (79.70 mmol/L) | 1.2g |
| Meglumine (19.93 mmol/L) | 0.1556g |
| Sodium chloride | 0.174g |
| Hydrochloric acid | Adjusting the pH to about 7.4 |
| Water for injection | Added to 40 mL |

### Preparation process:

1) Meglumine and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.2 mol/L hydrochloric acid solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Example 8

### Formula:

| | |
|---|---|
| Ketorolac tromethamine (79.70 mmol/L) | 1.2g |
| Citrate buffer (200 mmol/L, pH 7.4) | 0.008mol |
| Sodium hydroxide | Adjusting the pH to about 7.4 |
| Water | Added to 40 mL |

### Preparation process:

1) Ketorolac tromethamine was added to 40% formula amount of a citrate buffer (0.2 mol/L, pH 7.4), and the mixture was stirred until complete dissolution.
2) A 0.1 mol/L sodium hydroxide solution was added to adjust the pH value to about 7.4.
3) The citrate buffer (0.2 mol/L, pH 7.4) was supplemented to reach the formula amount.
4) The medicinal solution was filtered through a 0.22 µm polyethersulfone filter membrane and dispensed into clean vials at 1 mL/vial, and then the vials were plugged and capped.
5) The samples were subjected to moist heat sterilization at 121 °C for 15 min.
6) The sterilized products were labeled to give the final samples.

### Example 9

### Formula:

| | |
|---|---|
| Ketorolac tromethamine (79.70 mmol/L) | 1.2g |
| Acetate buffer (2000 mmol/L, pH 7.4) | 0.08mol |
| Sodium hydroxide | Adjusting the pH to about 7.4 |
| Water | Added to 40 mL |

### Preparation process:

1) Ketorolac tromethamine was added to 40% formula amount of an acetate buffer (2 mol/L, pH 7.4), and the mixture was stirred until complete dissolution.
2) A 0.1 mol/L sodium hydroxide solution was added to adjust the pH value to about 7.4.
3) The acetate buffer (2 mol/L, pH 7.4) was supplemented to reach the formula amount.
4) The medicinal solution was filtered through a 0.22 µm polyethersulfone filter membrane and dispensed into clean vials at 1 mL/vial, and then the vials were plugged and capped.
5) The samples were subjected to moist heat sterilization at 121 °C for 15 min.
6) The sterilized products were labeled to give the final samples.

### Comparative Example 1

### Formula:

| | |
|---|---|
| Ketorolac tromethamine | 1.2g |
| Sodium chloride | 0.174g |
| Sodium hydroxide | Adjusting the pH to about 7.4 |
| Water for injection | Added to 40 mL |

### Preparation process:

1) Sodium chloride was added to 85% formula amount of water for injection at room temperature, and the mixture was stirred at room temperature until complete dissolution.
2) Ketorolac tromethamine was added, and the mixture was stirred until complete dissolution.
3) A 0.1 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 2

### Formula:

| | |
|---|---|
| Ketorolac tromethamine | 0.6g |
| Polyoxyl 15 hydroxystearate | 0.8g |
| Sodium chloride | 0.087g |
| Sodium hydroxide | Adjusting the pH to about 7.4 |
| Water for injection | Added to 20 mL |

### Preparation process:

1) Polyoxyl 15 hydroxystearate and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.1 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 3

### Formula:

| | |
|---|---|
| Ketorolac tromethamine | 1.5g |
| Tween 80 | 2.5g |
| Sodium chloride | 0.2175g |
| Sodium hydroxide | Adjusting the pH to about 7.4 |
| Water for injection | Added to 50 mL |

### Preparation process:

1) Tween 80 and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.1 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 4

### Formula:

| | |
|---|---|
| Ketorolac tromethamine | 1.05g |
| Polyoxyl 35 castor oil (ELP) | 1.75g |
| Sodium chloride | 0.1523g |
| Sodium hydroxide | Adjusting the pH to about 7.4 |
| Water for injection | Added to 35 mL |

### Preparation process:

1) Polyoxyl 35 castor oil (ELP) and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.1 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 5

### Formula:

| | |
|---|---|
| Ketorolac tromethamine | 1.2g |
| Propylene glycol | 2g |
| Sodium chloride | 0.174g |
| Sodium hydroxide | Adjusting the pH to about 7.4 |
| Water for injection | Added to 40 mL |

### Preparation process:

1) Propylene glycol and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.1 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 6

### Formula:

| | |
|---|---|
| Ketorolac tromethamine | 1.2g |
| PEG 400 | 2g |
| Sodium chloride | 0.174g |
| Sodium hydroxide | Adjusting the pH to about 7.4 |
| Water for injection | Added to 40 mL |

### Preparation process:

1) PEG 400 and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.1 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 7

### Formula:

| | |
|---|---|
| Ketorolac tromethamine | 1.2g |
| EDTA | 0.044g |
| Sodium hydroxide | Adjusting the pH to about 7.4 |
| Water for injection | Added to 40 mL |

### Preparation process:

1) EDTA was added to 85% formula amount of water for injection at room temperature, and the mixture was stirred at room temperature until complete dissolution.
2) Ketorolac tromethamine was added, and the mixture was stirred until complete dissolution.
3) A 0.1 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 8

### Formula:

| | |
|---|---|
| Ketorolac tromethamine | 0.6g |
| Tromethamine | 0.0492g |
| Sodium chloride | 0.0806g |
| Sodium hydroxide | Adjusting the pH to about 7.4 |
| Water for injection | Added to 20 mL |

### Preparation process:

1) Tromethamine and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.2 mol/L hydrochloric acid solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 9

In accordance with the published information disclosed for a commercially available control, a sample with the strength of 1 mL: 15 mg was prepared using the same formula, and the specific preparation process is as follows:

### Formula:

| | |
|---|---|
| Ketorolac tromethamine | 0.3g |
| Ethanol | 2.0g |
| Sodium chloride | 0.1336g |
| Sodium hydroxide | Adjusting the pH to about 7.4 |
| Water for injection | Added to 20 mL |

### Preparation process:

1) Ethanol and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.5 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Comparative Example 10

In accordance with the published information disclosed for a commercially available control, a sample with the strength of 1 mL:30 mg was prepared using the same formula, and the specific preparation process is as follows:

### Formula:

| | |
|---|---|
| Ketorolac tromethamine | 0.6g |
| Ethanol | 2.0g |
| Sodium chloride | 0.087g |
| Sodium hydroxide | Adjusting the pH to about 7.4 |
| Water for injection | Added to 20 mL |

### Preparation process:

1) Ethanol and sodium chloride were added to 85% formula amount of water for injection at room temperature, and the mixture was stirred at room temperature until complete dissolution.
2) Ketorolac tromethamine was added, and the mixture was stirred until complete dissolution.
3) A 0.5 mol/L sodium hydroxide solution was added to adjust the pH value to about 7.4.
4) Water for injection was supplemented to reach the formula amount.
5) The medicinal solution was filtered through a 0.22 µm polyethersulfone filter membrane and dispensed into clean vials at 1 mL/vial, and then the vials were plugged and capped.
6) The samples were subjected to moist heat sterilization at 121 °C for 15 min.
7) The sterilized products were labeled to give the final samples.

### Example 10

### High-temperature test and illumination test:

The samples of Examples 1-9 and Comparative Examples 1-8 of the present disclosure, a 21265DK reference formulation (strength: 15 mg/mL; expiry date: 2022.09.01; manufacturer: Hospira, Inc.) and a 13084DK reference formulation (strength: 30 mg/mL; expiry date: 2022.01.01; manufacturer: Hospira, Inc.) were each placed under strong light irradiation (4500±500 Lx) at high temperature (60 °C) for 10 days. Sampling was performed on days 5 and 10, and the appearance properties, pH values, insoluble particles, related substances and contents of the resulting samples were tested. The test results were compared with the day-0 data of the samples in the same batch, as shown in Tables 1 and 2.

**Table 1. Results of high-temperature (60 °C) test**

| Sample | Time | Appearance property | pH value | Total impuritie s (%) | Content (%) | Number of insoluble particles | |
|---|---|---|---|---|---|---|---|
| | | | | | | 10 µm or greater | 25 µm or greater |
| Example 1 | Day 0 | Yellowish clear liquid | 7.43 | 0.14 | 99.0 | 33.7 | 1.7 |
| | Day 5 | Yellowish clear liquid | 7.40 | 0.13 | 99.5 | 35.0 | 0.0 |
| | Day 10 | Yellowish clear liquid | 7.38 | 0.06 | 98.7 | 313.3 | 5.0 |
| Example 2 | Day 0 | Colorless clear liquid | 7.60 | <0.05 | 100.5 | 35.0 | 0.0 |
| | Day 5 | Yellowish clear liquid | 7.60 | <0.05 | 100.2 | 20.0 | 0.0 |
| | Day 10 | Yellowish clear liquid | 7.50 | <0.05 | 101.2 | 11.7 | 0.0 |
| Example 3 | Day 0 | Yellowish clear liquid | 7.70 | 0.06 | 102.5 | 6.7 | 0.0 |
| | Day 5 | Yellowish clear liquid | 7.70 | 0.05 | 101.7 | 10.0 | 0.0 |
| | Day 10 | Yellowish clear liquid | 7.50 | 0.05 | 101.2 | 6.7 | 0.0 |
| Example 4 | Day 0 | Yellowish clear liquid | 7.41 | 0.12 | 98.6 | 3.3 | 0.0 |
| | Day 5 | Yellowish clear liquid | 7.38 | 0.12 | 98.8 | 1.7 | 0.0 |
| | Day 10 | Yellowish clear liquid | 7.39 | 0.06 | 98.9 | 8.3 | 0.0 |
| Example 5 | Day 0 | Clear (consistent with water) | 7.39 | 0.12 | 99.3 | 45.0 | 0.0 |
| | Day 5 | Clear (consistent with water) | 7.44 | 0.09 | 98.3 | 118.3 | 6.7 |
| | Day 10 | Clear (consistent with water) | 7.43 | 0.13 | 98.6 | 53.3 | 13.3 |
| Example 6 | Day 0 | Clear (between water and No. 0.5 turbidity standard) | 7.39 | 0.05 | 101.3 | 61.7 | 0.0 |
| | Day 5 | Clear (between water and No. 0.5 turbidity standard) | 7.42 | 0.12 | 103.2 | 463.3 | 33.3 |
| | Day 10 | Clear (between water and No. 0.5 turbidity standard) | 7.42 | 0.13 | 101.3 | 690.0 | 81.7 |
| Example 7 | Day 0 | Clear (between water and No. 0.5 turbidity standard) | 7.44 | 0.05 | 100.9 | 43.3 | 0.0 |
| | Day 5 | Clear (between water and No. 0.5 turbidity standard) | 7.45 | 0.10 | 102.2 | 345.0 | 18.3 |
| | Day 10 | Clear (between water and No. 0.5 turbidity standard) | 7.44 | 0.11 | 102.1 | 513.3 | 25.0 |
| Example 8 | Day 0 | Almost clear (between No. 0.5 and No. 1 turbidity standards) | 7.41 | 0.09 | 101.0 | 76.7 | 0.0 |
| | Day 5 | Clear (between water and No. 0.5 turbidity standard) | 7.43 | 0.08 | 100.0 | 113.3 | 0.0 |
| | Day 10 | Clear (between water and No. 0.5 turbidity standard) | 7.48 | 0.09 | 101.5 | 81.7 | 8.3 |
| Example 9 | Day 0 | Clear (between water and No. 0.5 turbidity standard) | 7.40 | 0.09 | 100.8 | 38.3 | 1.7 |
| | Day 5 | Clear (between water and No. 0.5 turbidity standard) | 7.45 | 0.12 | 99.0 | 248.3 | 6.7 |
| | Day 10 | Clear (between water and No. 0.5 turbidity standard) | 7.49 | 0.18 | 100.1 | 183.3 | 20.0 |
| Comparative Example 1 | Day 0 | Between No. 1 and No. 2 turbidity standards | 7.36 | 0.08 | 99.9 | 150.0 | 0.0 |
| | Day 5 | Almost clear (between No. 0.5 and No. 1 turbidity standards) | 7.42 | 0.09 | 102.8 | 496.7 | 60.0 |
| | Day 10 | Clear (between water and No. 0.5 turbidity standard) | 7.39 | 0.11 | 103.0 | 413.3 | 10.0 |
| Comparative Example 2 | Day 0 | Yellowish clear liquid | 7.39 | 0.16 | 103.4 | N/A | N/A |
| | Day 5 | Yellowish clear liquid | 7.24 | 0.22 | 97.6 | N/A | N/A |
| | Day 10 | Yellowish clear liquid | 7.23 | 0.28 | 99.2 | N/A | N/A |
| Comparative Example 3 | Day 0 | Yellowish clear liquid | 7.42 | 0.17 | 100.0 | 305.0 | 6.7 |
| | Day 5 | Yellow clear liquid | 7.41 | 0.51 | 101.7 | 80.0 | 0.0 |
| | Day 10 | Yellow clear liquid | 7.44 | 0.84 | 98.9 | 408.3 | 6.7 |
| Comparative Example 4 | Day 0 | Yellowish clear liquid | 7.36 | 0.21 | 100.4 | 51.7 | 0.0 |
| | Day 5 | Yellow clear liquid | 7.41 | 0.55 | 100.1 | 165.0 | 0.0 |
| | Day 10 | Yellow clear liquid | 7.35 | 0.76 | 98.3 | 518.3 | 3.3 |
| Comparative Example 5 | Day 0 | Between No. 1 and No. 2 turbidity standards | 7.41 | 0.11 | 101.8 | 128.3 | 5.0 |
| | Day 5 | Clear (between water and No. 0.5 turbidity standard) | 7.44 | 0.10 | 102.6 | 141.7 | 5.0 |
| | Day 10 | Clear (between water and No. 0.5 turbidity standard) | 7.43 | 0.10 | 102.8 | 70.0 | 5.0 |
| Comparative Example 6 | Day 0 | Almost clear (between No. 0.5 and No. 1 turbidity standards) | 7.40 | 0.15 | 101.6 | 520.0 | 11.7 |
| | Day 5 | Consistent with No. 1 turbidity standard | 7.44 | 0.19 | 101.8 | 485.0 | 33.3 |
| | Day 10 | Consistent with No. 1 turbidity standard | 7.41 | 0.20 | 102.6 | 201.7 | 18.3 |
| Comparative Example 7 | Day 0 | Clear (between water and No. 0.5 turbidity standard) | 7.42 | 0.08 | 102.0 | 70.0 | 0.0 |
| | Day 5 | Clear (between water and No. 0.5 turbidity standard) | 7.37 | 0.20 | 101.9 | 296.7 | 16.7 |
| | Day 10 | Consistent with No. 1 turbidity standard | 7.45 | 0.15 | 102.5 | 398.3 | 11.7 |
| Comparative Example 8 | Day 0 | Yellowish clear liquid | 7.36 | 0.15 | 102.1 | N/A | N/A |
| | Day 5 | Yellowish solution with a large number of white dots | 7.22 | 0.13 | 97.1 | N/A | N/A |
| | Day 10 | Yellowish solution with a large number of white dots | 7.20 | 0.15 | 102.4 | N/A | N/A |
| Reference (21265DK) | Day 0 | Clear, colorless | 7.35 | 0.10 | 101.9 | 17.3 | 0.0 |
| | Day 5 | Clear, colorless | 7.32 | 0.10 | 102.2 | 132.7 | 0.0 |
| | Day 10 | Clear, slightly yellowish-green | 7.26 | 0.10 | 102.0 | 45.0 | 0.0 |
| Reference (13084DK) | Day 0 | Clear, slightly yellowish-green | 7.35 | 0.09 | 102.3 | N/A | N/A |
| | Day 5 | Clear, slightly yellowish-green | 7.34 | 0.14 | 100.5 | N/A | N/A |
| | Day 10 | Clear, slightly yellowish-green | 7.31 | 0.19 | 101.2 | N/A | N/A |

**Table 2. Results of illumination (4500±500 Lx) test**

| Sample | Time | Appearance property | pH value | Total impurities (%) | Content (%) | Number of insoluble particles | |
|---|---|---|---|---|---|---|---|
| | | | | | | 10 µm or greater | 25 µm or greater |
| Example 1 | Day 0 | Yellowish clear liquid | 7.43 | 0.14 | 99.0 | 33.7 | 1.7 |
| | Day 5 | Yellowish clear liquid | 7.41 | 0.13 | 99.2 | 48.3 | 0.0 |
| | Day 10 | Yellowish clear liquid | 7.39 | 0.11 | 99.1 | 213.3 | 16.7 |
| Example 2 | Day 0 | Colorless clear liquid | 7.60 | <0.05 | 100.5 | 35.0 | 0.0 |
| | Day 5 | Yellowish clear liquid | 7.60 | <0.05 | 100.6 | 115.0 | 0.0 |
| | Day 10 | Yellowish clear liquid | 7.50 | <0.05 | 101.6 | 11.7 | 0.0 |
| Example 3 | Day 0 | Yellowish clear liquid | 7.70 | 0.06 | 102.5 | 6.7 | 0.0 |
| | Day 5 | Yellowish clear liquid | 7.70 | 0.05 | 102.1 | 11.7 | 0.0 |
| | Day 10 | Yellowish clear liquid | 7.50 | 0.05 | 102.3 | 8.3 | 0.0 |
| Example 4 | Day 0 | Yellowish clear liquid | 7.41 | 0.12 | 98.6 | 3.3 | 0.0 |
| | Day 5 | Yellowish clear liquid | 7.40 | 0.12 | 98.8 | 13.3 | 0.0 |
| | Day 10 | Yellowish clear liquid | 7.38 | 0.11 | 98.2 | 11.7 | 0.0 |
| Example 5 | Day 0 | Clear, colorless | 7.40 | 0.14 | 101.8 | N/A | N/A |
| | Day 5 | Clear, colorless | 7.34 | 0.17 | 102.6 | N/A | N/A |
| | Day 10 | Clear, colorless | 7.32 | 0.19 | 101.7 | N/A | N/A |
| Reference (21265DK) | Day 0 | Clear, colorless | 7.35 | 0.10 | 101.9 | 17.3 | 0.0 |
| | Day 5 | Clear, colorless | 7.29 | 0.10 | 101.9 | 301.3 | 0.0 |
| | Day 10 | Clear, slightly yellowish-green | 7.27 | 0.10 | 102.9 | 40.0 | 0.0 |
| Reference (13084DK) | Day 0 | Clear, slightly yellowish-green | 7.35 | 0.09 | 102.3 | N/A | N/A |
| | Day 5 | Clear, slightly yellowish-green | 7.34 | 0.14 | 100.6 | N/A | N/A |
| | Day 10 | Clear, slightly yellowish-green | 7.33 | 0.13 | 102.7 | N/A | N/A |

The results show that the samples of Examples 1-9 of the present disclosure were clear after sterilization, all of which complied with the provisions of the Chinese Pharmacopoeia (ChP), 2020 Edition, and the appearance properties, related substances, contents and pH values thereof did not change obviously after placement for 10 days under the conditions of high temperature (60 °C) and illumination; the clarity of the sterilized samples of Comparative Examples 1 and 5 did not comply with the provisions of the Chinese Pharmacopoeia (ChP), 2020 Edition; the sterilized samples of Comparative Examples 2, 3 and 4 were clear, but after placement for 10 days under the condition of high temperature (60 °C), the solutions had a significant change in appearance properties, appearing as yellow clear liquids, and had an obviously increased impurity content; and the sterilized samples of Comparative Examples 6, 7 and 8 after placement for 10 days under the condition of high temperature (60 °C) had appearance properties that did not comply with the provisions of the Chinese Pharmacopoeia (ChP), 2020 Edition, and had relatively poor stability.

### Example 11

### Accelerated test:

The samples of Examples 2 and 3 and a reference formulation (21265DK) were stored for 6 months in a constant temperature&humidity incubator at 40±2 °C and with 75%±5% relative humidity.

Sampling was performed in months 1, 2, 3 and 6 for testing, and the test results were compared with the day-0 data of the samples in the same batch, as shown in Table 3.

**Table 3. Results of accelerated test**

| Sample | Time | Appearance property | pH value | Total impurities (%) | Content (%) | Number of insoluble particles | |
|---|---|---|---|---|---|---|---|
| | | | | | | 10 µm or greater | 25 µm or greater |
| Example 2 | Month 0 | Colorless, clear | 7.5 | <0.05 | 103.9 | 35 | 0 |
| | Month 1 | Clear, slightly yellowish-green | N/A | <0.05 | 101.2 | N/A | N/A |
| | Month 2 | Clear, slightly yellowish-green | N/A | <0.05 | 102.7 | N/A | N/A |
| | Month 4 | Clear, slightly yellowish-green | N/A | <0.05 | 101.6 | N/A | N/A |
| | Month 6 | Clear, slightly yellowish-green | 7.6 | 0.05 | 101.3 | 15 | 0 |
| Reference (21265DK) | Month 0 | Clear, colorless | 7.35 | 0.10 | 101.9 | 17.3 | 0.0 |
| | Month 1 | Almost clear, slightly yellowish-green | 7.32 | 0.10 | 101.0 | 185.0 | 3.3 |
| | Month 2 | Clear, slightly yellowish-green | 7.31 | 0.12 | 100.1 | 126.7 | 0.0 |
| | Month 3 | Clear, slightly yellowish-green | 7.30 | 0.11 | 102.4 | N/A | N/A |
| | Month 6 | Clear, slightly yellowish-green | 7.27 | 0.11 | 101.7 | 45.0 | 10.0 |
| Example 3 | Month 0 | Yellowish clear liquid | 7.5 | 0.06 | 102.8 | 6.7 | 0.0 |
| | Month 1 | Yellowish clear liquid | N/A | <0.05 | 102.0 | N/A | N/A |
| | Month 2 | Yellowish clear liquid | N/A | 0.05 | 102.2 | N/A | N/A |
| | Month 4 | Yellowish clear liquid | N/A | 0.11 | 101.0 | N/A | N/A |
| | Month 6 | Yellowish clear liquid | 7.3 | 0.21 | 100.8 | 19 | 0 |
| Reference (15141DK) | Month 0 | Yellowish clear liquid | 7.30 | 0.23 | 99.3 | 5 | 0 |
| | Month 1 | Yellowish clear liquid | N/A | 0.23 | 100.6 | N/A | N/A |
| | Month 3 | Yellowish clear liquid | N/A | 0.24 | 99.6 | N/A | N/A |

The results of the accelerated test show that the contents of related substances of the samples of Examples 2 and 3, the reference formulation (21265DK, 1 mL: 15 mg) and the reference formulation (15141DK, 1 mL:30 mg) were slightly increased under the accelerated conditions, but were still within the range specified by the Chinese Pharmacopoeia (ChP), 2020 Edition, and the contents, appearance properties and pH values thereof did not change obviously, indicating that the samples of Examples 2 and 3 had good stability under the accelerated conditions.

### Example 12

### Long-term test:

The samples of Examples 2 and 3, a reference formulation (21265DK, 1 mL: 15 mg) and a reference formulation (15141DK, 1 mL:30 mg) were placed under the conditions of 25±2 °C and 60%±5% relative humidity for 24 months. Sampling was performed in months 3, 6, 9, 12, 18 and 24 for testing, and the test results were compared with the day-0 data of the samples in the same batch, as shown in Table 4.

**Table 4. Results of long-term test**

| Sample | Time | Appearance property | pH value | Total impurities (%) | Content (%) | Number of insoluble particles | |
|---|---|---|---|---|---|---|---|
| | | | | | | 10 µm or greater | 25 µm or greater |
| Example 2 | Month 0 | Clear, colorless | 7.5 | <0.05 | 103.9 | 35 | 0 |
| | Month 4 | Yellowish clear liquid | N/A | <0.05 | 100.6 | N/A | N/A |
| | Month 6 | Yellowish clear liquid | 7.6 | <0.05 | 102.2 | 2 | 0 |
| Reference (21265DK) | Month 0 | Clear, colorless | 7.35 | 0.10 | 101.9 | 17.3 | 0.0 |
| | Month 3 | Yellowish clear liquid | 7.28 | 0.11 | 102.5 | N/A | N/A |
| | Month 6 | Yellowish clear liquid | 7.29 | 0.12 | 101.8 | 13.3 | 1.7 |
| Example 3 | Month 0 | Yellowish clear liquid | 7.5 | <0.05 | 103.9 | 6.7 | 0.0 |
| | Month 4 | Yellowish clear liquid | N/A | 0.06 | 101.3 | N/A | N/A |
| | Month 6 | Yellowish clear liquid | 7.6 | 0.07 | 101.4 | 9 | 0 |
| Reference (15141DK) | Month 0 | Yellowish clear liquid | 7.30 | 0.23 | 99.3 | 5.0 | 0.0 |
| | Month 3 | Yellowish clear liquid | 7.34 | 0.22 | 100.1 | 37.0 | 4.0 |

The results of the long-term test show that the related substances and content changes of the samples of Examples 2 and 3 and the reference formulation (21265DK) were within the range specified by the Chinese Pharmacopoeia (ChP), 2020 Edition in a long term of 6 months, and the appearance properties and pH values of the samples did not change obviously, indicating that the samples of Examples 2 and 3 had good stability.

### Example 13

### Plasma protein binding assay:

The human plasma protein binding rates of the test samples (samples of Examples 2 and 3) were assayed by equilibrium dialysis and compared with the human plasma protein binding rate of a commercially available control (ketorolac tromethamine injection).

Two test samples (the sample of Example 2 with a strength of 1 mL: 15 mg and the sample of Example 3 with a strength of 1 mL:30 mg) and two commercially available controls (ketorolac tromethamine injections, one with a strength of 1 mL:15 mg and the other with a strength of 1 mL:30 mg, both manufactured by Hospira, Inc.) were each prepared into plasma samples with concentrations of 150 ng/mL, 1500 ng/mL and 15000 ng/mL, and the plasma samples were subjected to equilibrium dialysis at 37 °C for 6 h. Samples at the dosing and receiving ends and at the preparation instant (0 h) were assayed by LC-MS/MS, and the plasma protein binding (PPB) rates and the recovery rates were calculated based on the assay results. The specific data are shown in Table 5. The plasma samples and PBS samples prepared from the test samples and the commercially available controls were incubated at 37 °C for 6 h with oscillation (100 rpm) and then assayed for stability. The specific data are shown in Table 6. Meanwhile, a system control was set up, and the suitability of the test system was evaluated by determining the plasma protein binding rate of warfarin (300 ng/mL). The specific data are shown in Table 7.

**Table 5. Human plasma protein binding rates and recovery rates of test samples and commercially available controls**

| Assay substance (strength) | Concentration (ng/mL) | PPB (%) | | Recovery rate (%) | |
|---|---|---|---|---|---|
| | | Mean value | SD | Mean value | SD |
| Test sample (1 mL:15 mg) | 150 | 98.4 | 0.1 | 96.9 | 3.6 |
| | 1500 | 97.8 | 0.1 | 103.7 | 2.6 |
| | 15000 | 97.5 | 0.3 | 102.0 | 9.4 |
| Test sample (1 mL:30 mg) | 150 | 98.3 | 0.1 | 96.2 | 5.2 |
| | 1500 | 97.7 | 0.2 | 102.4 | 5.7 |
| | 15000 | 97.2 | 0.1 | 95.9 | 0.6 |
| Commercially available control (1 mL:15 mg) | 150 | 98.3 | 0.1 | 100.5 | 0.5 |
| | 1500 | 97.7 | 0.1 | 102.4 | 3.9 |
| | 15000 | 97.6 | 0.2 | 108.4 | 8.7 |
| Commercially available control (1 mL:30 mg) | 150 | 98.2 | 0.2 | 100.0 | 0.7 |
| | 1500 | 97.7 | 0.1 | 99.9 | 4.0 |
| | 15000 | 97.4 | 0.1 | 103.6 | 1.8 |

**Table 6. Stability of test samples and commercially available controls in human plasma and PBS**

| Assay substance (strength) | Substrate | Concentration (ng/mL) | Stability (%) | |
|---|---|---|---|---|
| | | | Mean value | SD |
| Test sample (1 mL:15 mg) | Human plasma | 150 | 96.0 | 0.8 |
| | | 1500 | 100.3 | 2.1 |
| | | 15000 | 96.1 | 1.4 |
| | PBS | 150 | 99.4 | 3.0 |
| | | 1500 | 97.5 | 2.1 |
| | | 15000 | 94.8 | 6.3 |
| Test sample (1 mL:30 mg) | Human | 150 | 97.9 | 5.2 |
| Assay substance (strength) | Substrate plasma | Concentration (ng/mL) | Stability (%) | |
| | | | Mean value | SD |
| | | 1500 | 105.0 | 0.7 |
| | | 15000 | 94.4 | 0.6 |
| | PBS | 150 | 102.7 | 6.0 |
| | | 1500 | 100.3 | 3.6 |
| | | 15000 | 98.4 | 1.0 |
| Commercially available control (1 mL:15 mg) | Human plasma | 150 | 94.7 | 2.7 |
| | | 1500 | 98.8 | 3.5 |
| | | 15000 | 104.2 | 7.9 |
| | PBS | 150 | 95.9 | 0.7 |
| | | 1500 | 104.7 | 2.8 |
| | | 15000 | 98.9 | 1.1 |
| Commercially available control (1 mL:30 mg) | Human plasma | 150 | 99.0 | 3.5 |
| | | 1500 | 99.9 | 3.2 |
| | | 15000 | 106.0 | 2.1 |
| | PBS | 150 | 105.4 | 1.2 |
| | | 1500 | 102.5 | 0.8 |
| | | 15000 | 105.1 | 1.1 |

**Table. 7. Human plasma protein binding rate and recovery rate of warfarin**

| Assay substance | Concentration (ng/mL) | PPB (%) | | Recovery rate (%) | |
|---|---|---|---|---|---|
| | | Mean value | SD | Mean value | SD |
| Warfarin | 300 | 99.1 | 0.0 | 96.2 | 3.1 |

### Results of plasma protein binding assay:

The human plasma protein binding rates of the system control warfarin (300 ng/mL) were both higher than 85%, indicating that the test system was suitable for this assay. At the test concentrations of 150 ng/mL, 1500 ng/mL and 15000 ng/mL, the human plasma protein binding rates of the test samples (the sample of Example 2 with a strength of 1 mL:15 mg and the sample of Example 3 with a strength of 1 mL:30 mg) were 97.2%-98.4% and the human plasma protein binding rates of the commercially available controls (ketorolac tromethamine injections with a strength of 1 mL:15 mg and 1 mL:30 mg, respectively) were 97.4%-98.3%, both of which exhibited high protein binding; no concentration dependence was found, and there was no difference in plasma protein binding rate between the test samples and the commercially available controls. The test samples and the commercially available controls were stable after incubation in human plasma and PBS solution at 37 °C for 6 h.

### Example 14

### In-vitro hemolysis assay:

With reference to the information on the clinical intended use of the ketorolac tromethamine injection, the concentrations for clinical intended use of the test samples (15 mg/mL and 30 mg/mL) were selected as the concentrations for this assay. Test sample 1 (the sample of Example 2 with a strength of 1 mL:15 mg), test sample 2 (the sample of Example 3 with a strength of 1 mL:30 mg), commercially available control 1 (strength: 1 mL:15 mg) and commercially available control 2 (strength: 1 mL:30 mg) with different volumes (0.5-0.1 mL) were separately added together with sodium chloride injections with different volumes (2.0-2.4 mL) into glass test tubes each containing 2.5 mL of a 2% rabbit erythrocyte suspension, and meanwhile, a sodium chloride injection and sterile water for injection were used as a negative control and a positive control, respectively, wherein the total volume of each test tube was 5.0 mL. The test tubes were incubated in an electric thermostat incubator at 37 °C for 3 h, and the lysis and coagulation of erythrocytes were observed. The specific assay method is shown in Table 8. The hemolysis and coagulation of the test samples and the commercially available controls after incubation in the 2% erythrocyte suspension for 15 min, 30 min, 45 min, 1 h, 2 h and 3 h were observed, and the results are shown in Table 9.

**Table 8. In-vitro hemolysis assay method**

| Test tube No. | 1 | 2 | 3 | | 4 | 5 | 6 | 7 | 8 | | 9 | 10 |
|---|---|---|---|---|---|---|---|---|---|---|---|---|
| 2% erythrocyte suspension (mL) | 2.5 | 2.5 | 2.5 | | 2.5 | 2.5 | 2.5 | 2.5 | 2.5 | | 2.5 | 2.5 |
| Sodium chloride injection (mL) | 2.0 | 2.1 | 2.2 | | 2.3 | 2.4 | 2.0 | 2.1 | 2.2 | | 2.3 | 2.4 |
| Test sample 1 (mL) | 0.5 | 0.4 | 0.3 | | 0.2 | 0.1 | - | - | - | | - | - |
| Commercially available control 1 (mL) | - | - | - | | - | - | 0.5 | 0.4 | 0.3 | | 0.2 | 0.1 |

| Test tube No. | 11 | 12 | 13 | 14 | 15 | 16 | 17 | 18 | 19 | 20 | 21 | 22 |
|---|---|---|---|---|---|---|---|---|---|---|---|---|
| 2% erythrocyte suspension (mL) | 2.5 | 2.5 | 2.5 | 2.5 | 2.5 | 2.5 | 2.5 | 2.5 | 2.5 | 2.5 | 2.5 | 2.5 |
| Sodium chloride injection (mL) | 2.0 | 2.1 | 2.2 | 2.3 | 2.4 | 2.0 | 2.1 | 2.2 | 2.3 | 2.4 | 2.5 | - |
| Test sample 2: (mL) | 0.5 | 0.4 | 0.3 | 0.2 | 0.1 | - | - | - | - | - | - | - |
| Commercially available control 2: (mL) | - | - | - | - | - | 0.5 | 0.4 | 0.3 | 0.2 | 0.1 | - | - |
| Sterile water for injection (mL) | - | - | - | - | - | - | - | - | - | - | - | 2.5 |

**Table 9. Observation results of hemolysis in each test tube at different times**

| Observation time | | | | | | | | Test tube No. | | | | | | | | |
|---|---|---|---|---|---|---|---|---|---|---|---|---|---|---|---|---|
| | 1 | 2 | | 3 | | 4 | | 5 | 6 | 7 | | 8 | | 9 | | 10 |
| | 2 | 4 | | | | | | | | | | | | | | |
| 15 min | - | - | | - | | - | | - | - | - | | - | | - | | - |
| 30 min | - | - | | - | | - | | - | - | - | | - | | - | | - |
| 45 min | - | - | | - | | - | | - | - | - | | - | | - | | - |
| 1 h | - | - | | - | | - | | - | - | - | | - | | - | | - |
| 2 h | - | - | | - | | - | | - | - | - | | - | | - | | - |
| Hemolysis after 3 h | No hemolysis | No hemolysis | | hemolysis | | No hemolysis | | hemolysis | hemolysis | No hemolysis | | hemolysis | | No hemolysis | | hemolysis |
| Coagulation after 3 h | No coagulation | No coagulation | | coagulation | | No coagulation | | coagulation | coagulation | No coagulation | | coagulation | | No coagulation | | coagulation |

| Observation time | | Test tube No. | | | | | | | | | | | | | | |
|---|---|---|---|---|---|---|---|---|---|---|---|---|---|---|---|---|
| | 11 | 12 | 13 | | 14 | | 15 | 16 | 17 | 18 | 19 | | 20 | | 21 | 22 |
| 15 min | | - | - | | - | | - | - | - | - | - | | - | | - | + |
| 30 min | | - | - | | - | | - | - | - | - | - | | - | | - | + |
| 45 min | | - | - | | - | | - | - | - | - | - | | - | | - | + |
| 1h | | - | - | | - | | - | - | - | - | - | | - | | - | + |
| 2h | | - | - | | - | | - | + | + | + | - | | - | | - | + |
| Hemolysis after 3 h | No hemolysis | No hemolysis | No hemolysis | | No hemolysis | | No hemolysis | No hemolysis | No hemolysis | No hemolysis | No hemolysis | | No hemolysis | | No hemolysis | Complete hemolysis |
| Coagulation after 3 h | No coagulation | No coagulation | No coagulation | | No coagulation | | No coagulation | No coagulation | No coagulation | No coagulation | No coagulation | | No coagulation | | No coagulation | / |

### Results of in-vitro hemolysis assay:

Test sample 1 (the sample of Example 2 with a strength of 1 mL:15 mg) and test sample 2 (the sample of Example 3 with a strength of 1 mL:30 mg) did not show hemolysis of rabbit erythrocytes *in vitro,* and neither caused coagulation of rabbit erythrocytes. The results of the test samples were consistent with those of the commercially available controls (ketorolac tromethamine injections).

### Example 15

### Active systemic anaphylaxis assay in guinea pigs:

Guinea pigs were sensitized by intramuscular injection of the test sample (the sample of Example 2 with a strength of 1 mL:15 mg) 3 times on days 1, 3 and 5. 14 days after the last sensitization administration, the first 3 animals in each group were challenged by intravenous injection into the feet. After the challenge, if neither the animals in the test sample groups (groups 3-4) nor the animals in the commercially available control group showed anaphylaxis symptoms, a secondary challenge was carried out on the remaining 6 animals in each group one week later (21 days after the last sensitization administration). The guinea pigs were observed for immediate hypersensitivity, and the observation results were compared with those of the commercially available control (ketorolac tromethamine injection with a strength of 1 mL:15 mg). The administration dose and administration volume for animals in each group are shown in Table 10; the evaluation criteria for sensitization are shown in Table 11; and the statistics of active systemic anaphylaxis in guinea pigs after administration with the test samples/controls are shown in Table 12.

**Table 10. Animal grouping, administration dose and administration volume**

| Group | Test sample/control | Animal No. | Sensitization (i.m.) At D1, D3 and D5 | | Challenge (i.v.) At D19/D19 and D26 | |
|---|---|---|---|---|---|---|
| | | | Administration dose mg/kg | Administration volume mL/kg | Administration dose mg/kg | Administration volume mL/kg |
| 1 | Negative control group (sodium chloride injection) | 1-9 | 0 | 1 | 0 | 1 |
| 2 | Positive control group (human serum albumin) | 10-18 | 40 | 1 | 80 | 1 |
| 3 | Low-dose test sample group | 19-27 | 2 | 1 | 4 | 1 |
| 4 | High-dose test sample group | 28-36 | 6 | 1 | 12 | 1 |
| 5 | Commercially available control group | 37-45 | 6 | 1 | 12 | 1 |

Sensitization evaluation was performed with reference to Table 11 below, and the results of the negative and positive control groups were comprehensively considered.

**Table 11. Evaluation criteria for systemic sensitization**

| Symptoms that appeared | Degree of anaphylaxis | Result judgment |
|---|---|---|
| 0 | - | Negative for anaphylaxis |
| 1-4 | + | Weak positive for anaphylaxis |
| 5-10 | ++ | Positive for anaphylaxis |
| 11-19 | +++ | Strong positive for anaphylaxis |
| 20 | ++++ | Extremely strong positive for anaphylaxis |

**Table 12. Statistics of active systemic anaphylaxis in guinea pigs after administration with test samples/controls**

| Group | Number of animals | Negative | Weak positive | Positive | Strong positive | Extremely strong positive |
|---|---|---|---|---|---|---|
| Negative control group | 9 | 9 | 0 | 0 | 0 | 0 |
| Positive control group | 9 | 0 | 0 | 0 | 4 | 5 |
| Low-dose test sample group | 9 | 9 | 0 | 0 | 0 | 0 |
| High-dose test sample group | 9 | 9 | 0 | 0 | 0 | 0 |
| Commercially available control group | 9 | 9 | 0 | 0 | 0 | 0 |

### Results of active systemic anaphylaxis assay in guinea pigs:

During the assay, the clinical observation of the animals in groups 1-5 before the challenge showed no abnormal reactions related to the test samples/commercially available control.

After the challenge, all of the 9 animals in the negative control group showed no anaphylaxis symptoms, being negative for anaphylaxis.

In the positive control group, 9 animals showed varying degrees of anaphylaxis symptoms, such as piloerection, nose scratching, cough, urination, dyspnea, purpura, gait instability, spasm, tidal breathing and the like, being strong positive to extremely strong positive for anaphylaxis, wherein 5 animals died.

All of the 9 animals in each of the low-dose test sample group, high-dose test sample group and commercially available control group showed no anaphylaxis symptoms, being negative for anaphylaxis.

Under the assay conditions, the test sample (the sample of Example 2 with a strength of 1 mL: 15 mg) was administered 3 times by intramuscular injections at doses of 2 mg/kg and 6 mg/kg for sensitization and was administered by intravenous injections at doses of 4 mg/kg and 12 mg/kg for challenge. 14 and 21 days after the last sensitization, no immediate hypersensitivity was observed in the guinea pigs. The results of the test samples were consistent with those of the commercially available control.

### Example 16

### Muscular irritation assay:

In this example, ketorolac tromethamine injection was injected intramuscularly into New Zealand rabbits once daily for a total of 5 doses, then the irritation responses of the muscles at the administration sites to the administration were observed, and the observation results were compared with those of a commercially available control.

16 male New Zealand rabbits weighing 2.55-3.08 kg were selected and randomly divided into 2 groups of 8. The animal grouping and administration concentrations are shown in Table 13. The left and right sides of the same body were used for self-control. A negative control (sodium chloride injection) was administered to all animals by intramuscular injection at the left quadriceps femoris muscle; a test sample (the sample of Example 3 with a strength of 1 mL:30 mg) at a concentration of 30 mg/mL was administered to animals in group 1 by intramuscular injection at the right quadriceps femoris muscle; and the commercially available control (ketorolac tromethamine injection) at a concentration of 30 mg/mL was administered to animals in group 2 by intramuscular injection at the right quadriceps femoris muscle. The administration volume was 0.5 mL/animal. The administration was carried out once daily for 5 days, namely D1-D5, and the day of the first administration was defined as D1. The first 3 animals in each group were euthanized on D8 (72±2 h after the last administration), and the remaining 5 animals in each group were euthanized on D19 (14 days after the last administration). The administration sites were kept and evaluated.

**Table 13. Animal grouping and administration concentrations**

| Group | Administration route | Administration site | Test sample /Control | Administration concentration (mg/mL) | Number of animals | Animal No. |
|---|---|---|---|---|---|---|
| 1 | Intramuscular injection | Left quadriceps femoris muscle | Negative control | 0 | 8 | 2192581-2192588 |
| | | Right quadriceps femoris muscle | Test sample | 30 | | |
| 2 | Intramuscular injection | Left quadriceps femoris muscle | Negative control | 0 | 8 | 2192589-2192596 |
| | | Right quadriceps femoris muscle | Commercially available control | 30 | | |

### Results of muscular irritation assay:

### Clinical observation:

During the assay, the animals showed no abnormal clinical manifestations related to the administration.

### Observation of administration sites

During the assay, the observation of the administration sites of the animals showed no abnormality related to the test sample/commercially available control.

### Gross pathology:

The animals were euthanized 72±2 h after administration (D8) and at the end of the recovery period (D19). For the animals in groups 1 and 2, gross observation showed a dark red discoloration at the right injection sites, and microscopic observation showed a mild interstitial hemorrhage at the related injection sites. The above-mentioned lesions were visible only at the right injection sites but not at the left injection sites, thus being considered to be related to the administered test sample and commercially available control. The results of the gross observation are detailed in Table 14.

**Table 14. Gross lesions related to test sample and commercially available control**

| **Gender** | | **Male** | |
|---|---|---|---|
| **GROUP** | | **1** | **2** |
| **Administration concentration (mg/mL)** | | **30** | **30** |
| **Type of death: euthanasia after administration** | | **3** | **3** |
| **Examination of the number of animals** | | | |
| **Injection site, right side** | | | |
| | Discoloration, dark red | 0 | 1 |
| **Type of death: euthanasia at the end of the recovery** period | | **5** | **5** |
| **Examination of the number of animals** | | | |
| **Injection site, right side** | | | |
| | Discoloration, dark red | 2 | 0 |

### Microscopic pathology:

The animals were euthanized 72±2 h after administration (D8). Microscopic observation of the right injection sites of the animals in groups 1 and 2 showed slight-to-moderate interstitial inflammatory cell infiltration, mild interstitial fibrosis, slight-to-mild interstitial hemorrhage, and slight-to-mild muscle fiber degeneration/necrosis with/without muscle fiber atrophy. The above-mentioned lesions were severe, had high incidence, and were visible only at the right injection sites but not at the left injection sites, thus being considered to be related to the administered test sample and commercially available control.

**Table 15. Microscopic lesions of animals euthanized after administration related to test sample/control**

| **Gender** | | **Male** | |
|---|---|---|---|
| **GROUP** | | **1** | **2** |
| **Administration concentration (mg/mL)** | | **30** | **30** |
| **Examination of the number of animals** | | **3** | **3** |
| **Injection site, right side** | | | |
| Infiltration, inflammatory cells; interstitium | | | |
| | Slight | 1 | 0 |
| | Mild | 0 | 1 |
| | Moderate | 0 | 1 |
| Fibrosis; interstitium | | | |
| | Mild | 1 | 2 |
| Hemorrhage; interstitium | | | |
| | Slight | 0 | 2 |
| | Mild | 0 | 1 |
| Degeneration/necrosis, muscle fiber, with/without muscle fiber atrophy | | | |
| | Slight | 1 | 1 |
| | Mild | 0 | 2 |

The animals were euthanized at the end of the recovery period (D19). The right injection sites of the animals in group 1 showed mild interstitial hemorrhage and slight-to-mild muscle fiber degeneration/necrosis with/without muscle fiber atrophy.

In addition, the right injection sites of the animals in group 2 showed slight-to-mild interstitial inflammatory cell infiltration, mild-to-moderate interstitial fibrosis, slight interstitial hemorrhage, mild-to-moderate muscle fiber degeneration/necrosis with/without muscle fiber atrophy, and sight mineralization.

The severity and incidence of lesions at the right injection sites of the animals euthanized at the end of the recovery period (D19) were not significantly reduced compared with those of the animals euthanized 72±2 h after administration (D8), suggesting that the above-mentioned lesions showed no recovery trend.

**Table 16. Microscopic lesions of animals euthanized at the end of recovery period related to test sample/control**

| **Gender** | | **Male** | |
|---|---|---|---|
| **GROUP** | | **1** | **2** |
| **Administration concentration (mg/mL)** | | **30** | **30** |
| **Examination of the number of animals** | | **5** | **5** |
| **Injection site, right side** | | | |
| Infiltration, inflammatory cells; interstitium | | | |
| | Slight | 0 | 1 |
| | Mild | 0 | 1 |
| Fibrosis; interstitium | | | |
| | Mild | 0 | 1 |
| | Moderate | 0 | 1 |
| Hemorrhage; interstitium | | | |
| | Slight | 0 | 3 |
| | Mild | 3 | 0 |
| Degeneration/necrosis, muscle fiber, with/without muscle fiber atrophy | | | |
| | Slight | 1 | 0 |
| | Mild | 2 | 1 |
| | Moderate | 0 | 2 |
| Mineralization | | | |
| | Slight | 0 | 1 |

Under the assay conditions, the ketorolac tromethamine injection was injected intramuscularly into the New Zealand rabbits once daily for 5 days. At the administration concentration of 30 mg/mL (the administration volume was 0.5 mL/animal), lesions related to the test sample and commercially available control were visible in the gross and microscopic observations of the injection sites. After a 14-day recovery period, the lesions at the injection sites showed no recovery trend. From the perspective of the lesions of the animals euthanized after the administration and at the end of the recovery period, the incidence and severity of the lesions at the right injection sites of the animals given the commercially available control were slightly greater than those of the animals given the test sample.

### Example 17

### Vascular irritation assay:

In this example, the ketorolac tromethamine injection was injected intravenously into New Zealand rabbits once daily for a total of 5 doses, then the irritation responses of the administration sites to the administration were observed, and the observation results were compared with those of a commercially available control.

16 male New Zealand rabbits weighing 2.74-3.74 kg were selected and randomly divided into 2 groups of 8. The animal grouping and administration concentrations are shown in Table 17. The left and right sides of the same body were used for self-control. A negative control (sodium chloride injection) was administered to all animals by injection into the left ear vein; a test sample (the sample of Example 2 with a strength of 1 mL:15 mg) was administered to animals in group 1 by injection into the right ear vein at an administration concentration of 15 mg/mL and an administration volume of 1 mL/animal; and the commercially available control (ketorolac tromethamine injection) was administered to animals in group 2 by injection into the right ear vein at an administration concentration of 15 mg/mL and an administration volume of 1 mL/animal.

The administration was carried out once daily for 5 days, namely D1-D5, and the day of the first administration was defined as D1. The first 3 animals in each group were euthanized on D8 (72±2 h after the last administration), and the remaining 5 animals in each group were euthanized on D19 (14 days after the last administration). The administration sites were kept and evaluated.

**Table 17. Animal grouping and administration concentrations**

| Group | Administration route | Administration site | Test sample /Control | Administration concentration (mg/mL) | Number of animals | Animal No. |
|---|---|---|---|---|---|---|
| 1 | Intravenous injection | Left ear vein | Negative control | 0 | 8 | 2192601∼2192608 |
| | | Right ear vein | Test sample | 15 | | |
| 2 | Intravenous injection | Left ear vein | Negative control | 0 | 8 | 2192609∼2192616 |
| | | Right ear vein | Commercially available control | 15 | | |

### Results of vascular irritation assay:

### Clinical observation:

During the assay, the animals showed no abnormal clinical manifestations related to the administration.

### Observation of administration sites:

During the assay, the observation of the administration sites of the animals showed no abnormality related to the test sample/commercially available control.

### Gross pathology:

During the assay, no abnormal pathological changes were visible in the gross observation of the injection sites (i.e., administration sites) of all animals euthanized 72 h (±2 h) after the last administration (D8) and 14 days after the last administration (D19).

### Microscopic pathology:

During the assay, no abnormal pathological changes were visible in the microscopic observation of bilateral five-segment veins at the injection sites (i.e., administration sites) of all animals euthanized 72 h (±2 h) after the last administration (D8) and 14 days after the last administration (D19).

The left administration site of 1 male animal in group 2 (#2192610) showed slight vascular wall necrosis and slight perivascular fibrosis. The above-mentioned lesions were seen in only 1 animal and were of low severity, thus being considered to be mechanical injuries and not related to the commercially available control.

Under the assay conditions, ketorolac tromethamine injection was repeatedly injected intravenously into the New Zealand rabbits at an administration concentration of 15 mg/mL and an administration volume of 1 mL/animal; and no abnormal pathological changes related to the test sample/commercially available control were visible in the gross and microscopic observations of the injection sites (i.e., tissues at administration sites) of the animals euthanized 72 h (±2 h) after the last administration (D8) and 14 days after the last administration (D19).

### Example 18

### Pharmacokinetics Study

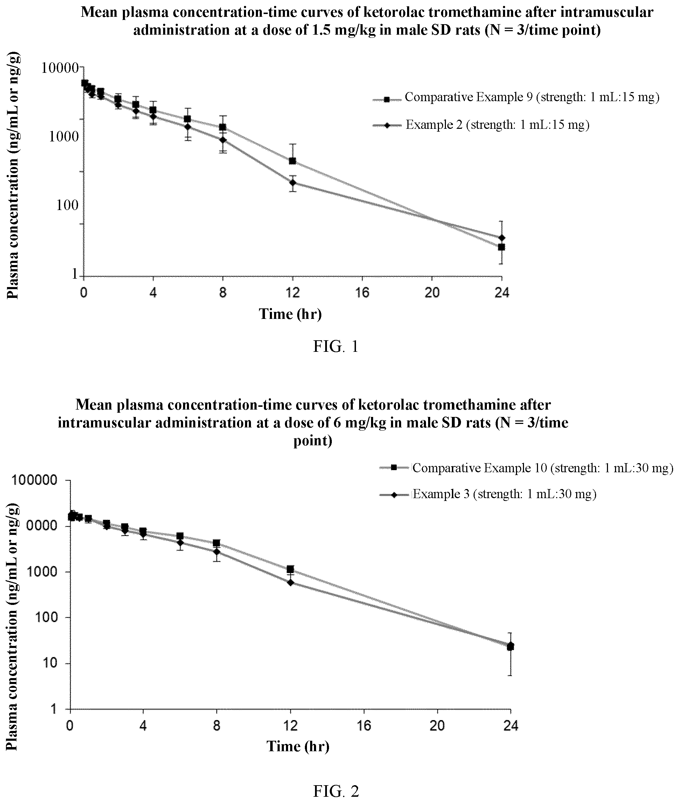

12 male SD rats were used in this experiment, and subjected to intravenous injection (1.5 mg/kg, using the sample of Example 2 with a strength of 1 mL: 15 mg and the sample of Comparative Example 9 with a strength of 1 mL: 15 mg) and intramuscular injection (6 mg/kg, using the sample of Example 3 with a strength of 1 mL:30 mg and the sample of Comparative Example 10) for a single dose. Plasma was collected 5 min, 15 min, 30 min, 1 h, 2 h, 3 h, 4 h, 6 h, 8 h, 12 h and 24 h after the administration. Then, the concentration of ketorolac tromethamine in rat plasma was quantitatively determined by an internal standard method using liquid chromatography with tandem mass spectrometry (LC-MS/MS) analysis, and pharmacokinetic parameters thereof were calculated by WinNonlin 8.2 software according to a non-compartmental model. The results are shown in Tables 18 and 19, and PK curves are shown in FIGs. 1 and 2.

**Table 18. Pharmacokinetics of ketorolac tromethamine in SD rats after single administration by intravenous injection using different ketorolac tromethamine formulations at 1.5 mg/kg**

| **PK parameters (unit)** | **Comparative Example 9 (strength: 1 mL:15 mg)** | **Example 2 (strength: 1 mL:15 mg)** |
|---|---|---|
| Cl (L/hr/kg) | 0.0991± 0.0487 | 0.1209±0.0258 |
| Vss (L/kg) | 0.319±0.0488 | 0.394± 0.0560 |
| T_{1/2}(hr) | 2.14± 0.107 | 2.43± 0.343 |
| AUCₗₐₛₜ(hr*ng/mL) | 17403± 7022 | 12732± 2455 |
| AUC_{INF}(hr*ng/mL) | 17414± 7027 | 12753± 2470 |
| MRT_{INF}(hr) | 3.57± 1.10 | 3.30 ±0.350 |
| MRTₗₐₛₜ(hr) | 3.55± 1.10 | 3.26± 0.319 |
| Vz (L/kg) | 0.304± 0.143 | 0.421± 0.0900 |

**Table 19. Pharmacokinetics of ketorolac tromethamine in SD rats after single administration by intramuscular injection using different ketorolac tromethamine formulations at 6 mg/kg**

| **PK parameters (unit)** | **Comparative Example 10 (strength: 1 mL:30 mg)** | **Example 3 (strength: 1 mL:30 mg)** |
|---|---|---|
| Tₘₐₓ(hr) | 0.194±0.0964 | 0.139±0.0964 |
| Cₘₐₓ(ng/mL) | 17133±1266 | 20033± 1904 |
| T _{1/2}(hr) | 2.15± 0.105 | 2.34± 0.158 |
| AUCₗₐₛₜ (hr*ng/mL) | 88468±6608 | 72637±17829 |
| AUC_{INF} (hr*ng/mL) | 88540±6614 | 72727± 17905 |
| MRT_{INF}(hr) | 4.49±0.218 | 3.77± 0.660 |
| MRTₗₐₛₜ(hr) | 4.47± 0.22 | 3.74± 0.646 |
| Vz_F (L/kg) | 0.211± 0.0217 | 0.286± 0.0467 |
| Cl_F (L/ hr /kg) | 0.068± 0.0053 | 0.0856± 0.0192 |

The above assay results show that the ketorolac liquid composition of the present disclosure can achieve excellent exposure, but has the advantages of significantly reduced *in-vivo* irritation, fewer adverse effects and excellent safety. In addition, the ketorolac liquid composition is more convenient for medication, and can improve medication compliance of patients and the convenience of clinical medication.

## Claims

1. A ketorolac liquid composition, comprising the following components: ketorolac or a pharmaceutically acceptable salt thereof, a stabilizer, an osmolarity regulator, a pH regulator, and optionally a buffer present or absent,
wherein the ketorolac liquid composition does not comprise ethanol; a concentration of ketorolac or the pharmaceutically acceptable salt thereof is 0.15 mmol/L-160.00 mmol/L, and the concentration refers to the ratio of the molar amount of ketorolac or the pharmaceutically acceptable salt thereof to the volume of the ketorolac liquid composition;
preferably, in the ketorolac liquid composition, the pharmaceutically acceptable salt of ketorolac is ketorolac tromethamine;
preferably, in the ketorolac liquid composition, the concentration of ketorolac or the pharmaceutically acceptable salt thereof (e.g., ketorolac tromethamine) is 0.15 mmol/L-0.38 mmol/L, or 30.00 mmol/L-160.00 mmol/L, such as 39.50 mmol/L-80.00 mmol/L.

2. The ketorolac liquid composition according to claim 1, wherein: in the ketorolac liquid composition, the stabilizer is one or more selected from lysine, arginine, meglumine, phosphoric acid, hydroxypropyl-β-cyclodextrin and sulfobutyl-β-cyclodextrin, and preferably one or more selected from arginine, meglumine, hydroxypropyl-β-cyclodextrin and sulfobutyl-β-cyclodextrin;
preferably, the lysine is L-lysine and/or D-lysine;
preferably, the arginine is L-arginine and/or D-arginine.

3. The ketorolac liquid composition according to claim 1 or 2, wherein: in the ketorolac liquid composition, the molar ratio of ketorolac or the pharmaceutically acceptable salt thereof to the stabilizer is 1:0.005-1:30, and preferably 1:0.05-1:5.

4. The ketorolac liquid composition according to any one of claims 1 to 3, wherein: in the ketorolac liquid composition, the buffer is one or more selected from acetic acid and a salt thereof, boric acid and a salt thereof, citric acid and a salt thereof, tartaric acid and a salt thereof, oxalic acid and a salt thereof, carbonic acid and a salt thereof, and tromethamine and a salt thereof, and preferably one or more selected from citric acid and a salt thereof and acetic acid and a salt thereof; preferably, the salt is a sodium salt, a potassium salt, a calcium salt or an ammonia salt.

5. The ketorolac liquid composition according to any one of claims 1 to 4, wherein: in the ketorolac liquid composition, the molar ratio of ketorolac or the pharmaceutically acceptable salt thereof to the buffer is 1:0-1:30, and preferably 1:0-1:5.

6. The ketorolac liquid composition according to any one of claims 1 to 5, wherein: in the ketorolac liquid composition, the pH regulator is one or more selected from hydrochloric acid, sulfuric acid, acetic acid, citric acid, sodium hydroxide, sodium bicarbonate and aqueous ammonia; preferably, the pH of the ketorolac liquid composition is in a range of 6.0-9.0, and further preferably in a range of 6.5-8.5.

7. The ketorolac liquid composition according to any one of claims 1 to 6, wherein: in the ketorolac liquid composition, the osmolarity regulator is one or more selected from sodium chloride, mannitol, glucose and lactose;
preferably, the osmolality of the ketorolac liquid composition is in a range of 200 mOsmol/kg-500 mOsmol/kg, and further preferably in a range of 260 mOsmol/kg-330 mOsmol/kg.

8. The ketorolac liquid composition according to any one of claims 1 to 7, wherein: the ketorolac liquid composition further comprises water, such as water for injection;
preferably, the ketorolac liquid composition may be an oral liquid or an injection.

9. A preparation method for the ketorolac liquid composition according to any one of claims 1 to 8, comprising mixing the components according to any one of claims 1 to 8.

10. Use of the ketorolac liquid composition according to any one of claims 1 to 8 for manufacturing a medicament for the prevention and/or treatment of inflammation, such as for manufacturing an anti-inflammatory medicament, preferably a non-steroidal anti-inflammatory medicament.
